# EUROPEAN PATENT SPECIFICATION

(11) **EP 0 750 504 B1**
(45) Date of publication and mention of the grant of the patent: **19.06.2002**
(21) Application number: 95912926.3
(22) Date of filing: 14.03.1995
(51) Int. Cl.: A61K 31/715, A61P 3/10

(54) **USE OF COMPLEX CARBOHYDRATE TO DIMINISH HYPOGLYCEMIA IN PATIENTS WITH DIABETES MELLITUS**
VERWENDUNG EINES KOMPLEXES KOHLENHYDRATES ZUR VERRINGERUNG DER HYPOGLYKÖMIE BEI DIABETES MELLITUS-PATIENTEN
UTILISATION D'UN GLUCIDE COMPLEXE POUR REDUIRE L'HYPOGLYCEMIE CHEZ LES PATIENTS SOUFFRANT DE DIABETE SUCRE

(30) Priority: 15.03.1994 US 213542
(43) Date of publication of application: 02.01.1997
(73) Proprietor: CHILDREN'S HOSPITAL OF LOS ANGELES, Los Angeles, CA 90054-0700 (US)
(72) Inventor: KAUFMAN, Francine, Los Angeles, CA 90049 (US)
(74) Representative: Ritter, Stephen David
(86) International application number: US9503330
(87) International publication number: WO9524906

(56) References cited:
- AM.J.CLIN.NUTR., vol. 47, no. 3, 1988 pages 428-32, 'Effect of stach structure on glucose and insulin responses in adults'
- AM.J.CLIN.NUTR., vol. 42, no. 3, 1985 pages 462-9,
- AM.J.CLIN.NUTR., vol. 56, no. 3, 1992 pages 587-92, 'Optimal daytime feding regimen to prevent postprandial hypoglycemia in type 1 glycogen storage disease'
- AM.J.CLIN.NUTR., vol. 52, no. 4, 1990 pages 667-70, 'Cornstarch ingestion after oral glucose: effect on glucose concentrations, hormone responses, and symptoms in patients with postprandial hypoglycemic syndrome'
- AM.J.CLIN.NUTR., vol. 47, no. 6, 1988 pages 1001-3,
- EUR.J.CLIN.NUTR., vol. 47, no. 4, 1993 pages 268-73, 'Complex carbohydrates in the prevention of noctural hypoglycemia in diabetic children' cited in the application
- AM.J.CLIN.NUTR., vol. 52, 1990 pages 1051-7, 'Physical growth and development of chidren with type 1 glycogen-storage disease: comparison of the effects of long term use of dextrose and uncooked cornstarch' cited in the application
- J.INVEST.MED., vol. 43(suppl.1), 1995 page 188A. 'A randomised crossover, blinded trial of uncooked cornstarch to diminish noctural hypoglycemia at diabetes camp'

## Description

### BACKGROUND OF THE INVENTION

### 1. Field of the Invention

This invention relates to therapeutic treatments of diabetes mellitus. More particularly, this invention relates to the use of uncooked cornstarch in the treatment of hypoglycemia caused by Type I and Type II diabetes mellitus.

### 2. Description of Related Art

Symptoms of hypoglycemia fall into two main categories. Rapid epinephrine release causes sweating, tremor, tachycardia, anxiety, and hunger. Central nervous system symptoms include dizziness, headache, clouding of vision, blunted mental acuity, confusion, abnormal behavior, convulsions, and loss of consciousness. When hypoglycemia is recurrent or severe, nervous system symptoms predominate, and the epinephrine phase may not be recognizable. With more rapid drops or wide swings in plasma glucose (as in insulin reactions), adrenergic symptoms are prominent (*Harrison's Principles of Intemal Medicine*, 11th Ed., McGraw-Hill Book Company, New York, 1987, p. 1800).

Numerous strategies have been developed to achieve the goal of maintaining blood glucose at a relatively constant level, such as open looped continuous subcutaneous insulin pumps and multiple daily injections of insulin. These intensive insulin regimens are coupled with home glucose monitoring, and many patients measure their blood glucose levels by finger prick up to 6 to 8 times per day to assure that close to normal blood sugar levels are maintained. This regimen is prescribed because studies have shown that by avoiding excessive high blood sugar levels, the long-term outcome of patients with diabetes can be improved. However, this regimen, which decreases episodes of high blood sugar, also causes patients to experience more low blood sugar reactions (hypoglycemia). Results of the Diabetes Complication and Control Trial indicate that intensive insulin treatment, while it markedly delays and lessens long term retinal, nephrologic and neuropathic disease, leads to a three to nine-fold increase in hypoglycemic events, most of which occur at night (L.Y. Dawson, *Clinical Diabetes,* 11:88-96, 1993). Sometimes these episodes of hypoglycemia are severe and can lead to loss of consciousness and convulsions. Severe hypoglycemic events seem to occur more often at night while the patient is asleep rather than during the day. When awake, diabetic patients can feel hypoglycemic reactions beginning, and can treat themselves with sugar in order to bring their blood sugar levels back into the normal range. When asleep, patients do not have this awareness, therefore the risk of hypoglycemic is much higher during this time.

The need exists to develop strategies to diminish hypoglycemia while continuing to intensively manage diabetes. Cornstarch has been used effectively to combat the hypoglycemia associated with glycogen storage disease type 1, a disease having an inherited absence or deficiency of glucose-6-phosphatase activity in the liver, kidney, and intestines, leading to accumulation of glycogen in those organs and hypoglycemia during fasting. Protection against low blood sugar was provided for up to 6 to 8 hours after ingestion of uncooked cornstarch (J. I. Wolfsdorf, et *al., Am. J. Clin. Nutr.,* 52:1051-7, 1990). However, the dosage of cornstarch used for this treatment was 1.75 grams per kilogram of body weight. This dosage is much higher than could be tolerated by a patient with diabetes mellitus.

Another study has also been conducted in patients with diabetes, giving cornstarch during inpatient hospitalization, with a reduction in the nadir of the blood glucose level. Children were fasted and then given the entire carbohydrate content of the standard bedtime snack (30 grams of carbohydrate) as uncooked cornstarch (M.T. Ververs, *et al., Eur. J. Clin. Nutr.*, 47:268-73, 1983). However, this study did little to prevent hypoglycemia and did not evaluate varying dosages to determine maximal efficacy.

*Am. J. Clin. Nutr.* 1990 52, 667-670 refers to a study wherein 75 g of raw cornstarch is diluted with water to a volume of 240 ml and is ingested following the ingestion of 75 g oral glucose. However, no attempt was made in this study to evaluate the effect of varying dosages.

*Am. J. Clin. Nutr.* 1992 56, 587-592 refers to a study to determine the optimal daytime dietary regime for type I glycogen storage disease, using a breakfast mixed meal consisting of cereal and uncooked cornstarch. The uncooked cornstarch is given in a slurry of cold water.

*Am. J. Clin. Nutr.* 1985 42, 462-469 refers to a study in the differences on the responses of nondiabetics and diabetics to food physical factors. A reference meal comprising uncooked flummery which consists of cornstarch protein and fat to match the macronutrient content of the "bean" meals under study. This study was not directed at the prevention or reduction of episodes of hypoglycemia in diabetics and thus no attempts were made to study the optimum dosage range for regulating and stabilizing blood glucose levels in diabetics.

Thus, the need exists for a better method of treating hypoglycemia in diabetics whether it results from too large a dose of insulin in patients with Type II diabetes who use insulin, or whether it occurs in patients with Type I diabetes, such as in pediatric patients during sleep.

### SUMMARY OF THE INVENTION

According to one aspect of the present invention there is provided the use of a complex carbohydrate in the manufacture of a pharmaceutical composition for regulating and stabilizing blood glucose levels in humans with Diabetes Mellitus characterised in that the pharmaceutical composition is in the form of a snack bar, ingestion of which provides said complex carbohydrate in the dose of 0.1 to 1.0 gram per kilogram of body weight.

The invention further provides a pharmaceutical composition being a unit dose preparation of uncooked cornstarch in the form of a snack bar.

### A DETAILED DESCRIPTION OF THE INVENTION

This study will attempt to determine if uncooked cornstarch, given in a dose of 1.0 gram per kilo added to milk (equivalent to 25-50% of the evening snack carbohydrate calories) can diminish the incidence and severity of hypoglycemia in hospitalized patients receiving subcutaneous insulin and in outpatients while on their usual insulin regimens.

This invention provides a method of utilizing complex carbohydrates, and in particular, uncooked cornstarch, to stabilize glucose levels in patients by titrating the dosage so as to avoid both hyper- and hypoglycemia, for in diabetics hyperglycemia can be caused by ingestion of too much carbohydrate. In particular, this invention provides a method for administering to a diabetic patient a measured dose of complex carbohydrate, preferably in the form of uncooked cornstarch, to stabilize blood sugar levels above that generally identified with the onset of clinical symptoms of hypoglycemia. The definition of the onset of clinical symptoms of hypoglycemia as used herein is a blood glucose level less than or equal to 3.0 mmol/liter or 60 mg/deciliter of whole blood.

In the practice of this invention, uncooked cornstarch is the preferred source of complex carbohydrate since its carbohydrate content is relatively uniform, its rate of metabolism is both known and uniform, and can be readily formulated into snack bars.

Diabetic patients cannot tolerate the high dosage of uncooked comstarch used in the treatment of glycogen storage disease without developing hyperglycemia. The preferred dose of complex carbohydrate administered to a diabetic patient to forestall development of the clinical symptoms of hypoglycemia is, therefore, much lower, about 0.1 to 1.0 gram per kilogram, more preferably about 0.25 to 0.5 grams per kilogram of body weight.

In addition, in children under the age of two years, it is also preferred that the dosage of complex carbohydrate be administered together with the enzyme pancrease in an amount useful to promote digestion of complex carbohydrate. The pancrease is given adjunctively, usually in a dosage of about 1/4 teaspoon of pancrease per dose of complex carbohydrate.

All patients taking insulin to facilitate post-prandial absorption of glucose can be treated recurrently during the day with premeasured doses of complex carbohydrate that is slowly metabolized to the monosaccharide glucose over a period of six to eight hours instead of being treated with simple carbohydrates, such as orange juice or other sugar sources that tend to cause a rapid peak in blood glucose level that quickly subsides. During waking hours the patient's use of, and hence requirement for, glucose is varied and depends upon the level and type of activity. The exact amount and frequency of the actual dose, therefore, will vary by patient and from day to day for each patient. A blood glucose test, usually administered as a finger prick to obtain a blood sample, can be used to monitor daily glucose levels as well as the patient's own subjective experience of symptoms associated with the onset of hypoglycemia. Therefore, in the practice of this invention sufficient complex carbohydrate is administered, preferably in the form of uncooked cornstarch, to maintain the blood glucose level somewhat above this level.

To prevent hypoglycemia during overnight sleep periods, the complex carbohydrate is administered to the patient at bedtime, and the above described dose of uncooked cornstarch is sufficient so the patient passes the nocturnal sleep time without need for glucose intervention and awakens after about six to eight hours of sleep with a glucose level of 60 mg/dl or greater. Many diabetics routinely consume a standard bedtime snack containing about 30 grams of carbohydrate, such as bread, or cereal and milk. In the practice of this invention, about 1/4 to 1//2 of the bedtime carbohydrate snack is replaced by uncooked cornstarch to stabilize blood sugar levels through the night (for six to eight hours) and to wear off in time for the patient to awaken with a normal blood sugar level.

For convenience in measuring the dosage actually consumed, it is preferred that the complex carbohydrate be prepared in unit dosage forms of 5 grams each. To assure proper metabolism of the complex carbohydrate, it is also preferred that the it be consumed together with at least two other food exchanges selected from the group consisting of fat, protein and other carbohydrates. The preferred complex carbohydrate is cornstarch, which is routinely used as a binder in formulation of tablets (see *Remington's Pharmaceutical Sciences,* 18th Edition, Mack Publishing Company, 1990, pp. 1633-1675). Ingredients in addition to uncooked cornstarch used in the formulation may be inert to metabolism, such as cellulosic binders and the like. If not inert, the nutritional contribution of such formulation ingredients to the carbohydrate content of the unit dosage shared be taken into account in formulating the unit dosage. Preferably, the uncooked cornstarch is incorporated into a snack bar containing a total of about 30 grams of carbohydrate (comparable to prior art bedtime snacks for diabetics), but having about 1/2 to 1/4 of the carbohydrate in the snack being uncooked comstarch in the form of a filling or coating, for example, one sweetened with a non-sugar sweetener.

The following examples illustrate the manner in which the invention can be practiced.

### EXAMPLE 1

Forty patients with Type I Diabetes Mellitus who were receiving subcutaneous insulin hospitalized at Children's Hospital of Los Angeles or were seen in the Endocrine Outpatient Clinic and were receiving their usual insulin regimens were asked to participate in a study to determine whether administration of uncooked cornstarch as part of the evening snack would diminish the incidence and severity of hypoglycemia. On day one, ten patients, 2 to 25 years of age were treated as inpatients and patients received 1.0 gram/kg of body weight of uncooked comstarch (Arrow Cornstarch) in milk as part of the bedtime snack. Hourly bedside blood glucose determinations were done from midnight until 6 a.m. by fingerstick requiring a single drop of blood. In addition, observable symptoms of hypoglycemia, such as thrashing about or convulsions were recorded. If significant hyperglycemia was experienced, the dosage of uncooked comstarch for day two was reduced to 0.5 g/kg. If on the other hand, significant hypoglycemia was experienced, on day two the dose was increased to 1.5 grams/kg. In addition, an oral report of the patient's observable nighttime symptoms was made either by the patient or its parent to an attending physician at least three times per week to monitor the patient's progress on the study. Data was compared to inpatient records for the last two years and matched by age, sex and duration of diabetes.

Patients treated as inpatients of Children's Hospital Los Angeles have a significant reduction in middle of the night hypoglycemia while receiving the cornstarch-containing snack compared to the regular snack. There was no significant elevation of the blood glucose level after ingestion of the comstarch-containing snack as would be seen with simple carbohydrates.

Thirty patients, 2 to 25 years of age, were tested as outpatients. Blood sugar tests were conducted prior to bed, at 2 to 3 a.m., and in the morning when awakened. The three blood sugar level readings were recorded for a two week period during which the patient received the standard bedtime snack containing 30 grams of carbohydrate containing no cornstarch. During the following two week period, the same regimen was followed except that 25 to 50% of the carbohydrate portion of the evening snack administered at bedtime was uncooked cornstarch. Once again blood sugar levels were measured and recorded just prior to bed, at 2 to 3 a.m., and in the morning when awakened.

Blood glucose levels obtained during the first two week period were compared with those obtained during the second two week period. Statistical analysis will be performed with student t-test for inter and intra group comparisons for both blood sugar levels and incidence of symptomatic hypoglycemic events.

### EXAMPLE 2

Twenty patients with a history of nighttime hypoglycemia, ages 5-16 years, and having had diabetes mellitus for more than one year, participated in the following study. For the first two week period the patients received the standard bedtime snack containing 30 grams of carbohydrate but no uncooked cornstarch. Blood glucose readings were done at 2 a.m. and before breakfast, with an at home glucose meter. During the second two week period, 1/4 to 1/2 of the carbohydrate content of the bedtime snack for each patient was given as uncooked cornstarch in milk. In Table 1 below the mean (±SD) number of hypoglycemic episodes for the 13 subjects (as characterized by a blood glucose reading less than 60 mg/dl) are shown for the 2 a.m. reading and the before breakfast readings.

**TABLE 1**

| | **14 days Standard** | **14 days Cornstarch** |
|---|---|---|
| 2 a.m. | 2.00 ± 2.12 | 0.61 ± 0.87 |
| before breakfast | 2.61 ± 2.25 | 0.69 ± 1.03 |

Our results suggest that uncooked cornstarch can be used to decrease episodes of nocturnal hypoglycemia in young patients with diabetes mellitus and history of hypoglycemia. This was accomplished without increasing the mean AM blood glucose level, therefore not altering overall glycemic control.

## Claims

1. The use of a complex carbohydrate in the manufacture of a pharmaceutical composition for regulating and stabilizing blood glucose levels in humans with Diabetes Mellitus **characterised in that** the pharmaceutical composition is in the form of a snack bar, ingestion of which provides said complex carbohydrate in the dose of 0.1 to 1.0 gram per kilogram of body weight.

2. The use of Claim 1 wherein the complex carbohydrate is uncooked cornstarch.

3. The use of Claim 1 or Claim 2 wherein the dose is from 0.25 to 0.5 grams per kilogram of body weight.

4. The use of any preceding claim wherein the Diabetes Mellitus is Type I and the human further receives a regimen of subcutaneous insulin therapy.

5. The use of any of Claims 1 to 3 wherein the Diabetes Mellitus is Type II and the human further receives a regimen of subcutaneous insulin therapy.

6. The use of any preceding claim wherein the snack bar contains 30 grams of carbohydrate and uncooked cornstarch is substituted for from ¼ to ½ of the carbohydrate contained therein.

7. The use of Claim 6 wherein the uncooked cornstarch is contained within a filling for or coating upon the snack bar and is sweetened with a non-sugar sweetener.

8. The use of any preceding claim wherein the uncooked cornstarch is formulated in 5 gram unit doses.

9. The use of any preceding claim wherein the unit doses are formulated for sustained release.

10. The use of any preceding claim wherein the blood glucose level is stabilized to 60 mg/dl or greater for at least six hours.

## Patentansprüche

1. Verwendung eines komplexen Carbohydrats bei der Herstellung einer pharmazeutischen Zubereitung zum Regulieren und Stabilisieren von Blutglukosespiegeln bei Menschen mit Diabetes Mellitus, **dadurch gekennzeichnet, dass** die pharmazeutische Zubereitung in Form eines Imbissriegels (snack bar) vorliegt, dessen Aufnahme das komplexe Kohlenhydrat in der Dosis von 0,1 bis 1,0 g pro kg Körpergewicht bereitstellt.

2. Verwendung gemäß Anspruch 1, worin das komplexe Kohlenhydrat nicht gekochte Maisstärke ist.

3. Verwendung gemäß Anspruch 1 oder Anspruch 2, worin die Dosis von 0,25 bis 0,5 g pro kg Körpergewicht beträgt.

4. Verwendung gemäß einem der vorstehenden Ansprüche, worin der Diabetes Mellitus der Typ I ist und der Mensch zusätzlich die Maßnahme der subkutanen Insulintherapie erhält.

5. Verwendung gemäß einem der Ansprüche 1 bis 3, worin der Diabetes Mellitus der Typ II ist und der Mensch zusätzlich die Maßnahme der subkutanen Insulintherapie erhält.

6. Verwendung gemäß einem der vorstehenden Ansprüche, worin der Imbissriegel 30 g Kohlenhydrat enthält und ungekochte Maisstärke von einem Viertel (1/4) bis zur Hälfte (1/2) des darin enthaltenen Kohlenhydrats ersetzt.

7. Verwendung gemäß Anspruch 6, worin die ungekochte Maisstärke in einer Füllung für oder eine Beschichtung auf dem Imbissriegel enthalten ist und mit einem Nicht-Zucker-Süßungsmittel gesüßt wird.

8. Verwendung gemäß einem der vorstehenden Ansprüche, worin die nicht gekochte Maisstärke in Einheitsdosen von 5 g formuliert ist.

9. Verwendung gemäß einem der vorstehenden Ansprüche, worin die Einheitsdosen für die verzögerte Freisetzung formuliert sind.

10. Verwendung gemäß einem der vorstehenden Ansprüche, worin der Blutglukosespiegel auf 60 mg/dl oder darüber für mindestens sechs Stunden stabilisiert wird.

## Revendications

1. Utilisation d'un hydrate de carbone complexe dans la préparation d'une composition pharmaceutique destinée à la régulation et à la stabilisation des taux de glucose sanguins chez un individu humain atteint de diabète sucré, **caractérisée en ce que** la composition pharmaceutique est sous forme d'une barre à croquer dont l'ingestion fournit ledit hydrate de carbone complexe à une dose de 0,1 à 1,0 g par kg de masse corporelle.

2. Utilisation selon la revendication 1, dans laquelle l'hydrate de carbone complexe est de l'amidon de maïs cru.

3. Utilisation selon la revendication 1 ou la revendication 2, dans laquelle la dose est comprise entre 0,25 et 0,5 g par kg de masse corporelle.

4. Utilisation selon l'une quelconque des revendications précédentes, dans laquelle le diabète sucré est de type I et l'individu humain est soumis en outre à un traitement par de l'insuline sous-cutanée.

5. Utilisation selon l'une quelconque des revendications 1 à 3, dans laquelle le diabète sucré est de type II et l'individu humain est soumis en outre à un traitement par de l'insuline sous-cutanée.

6. Utilisation selon l'une quelconque des revendications précédentes, dans laquelle la barre à croquer contient 30 g d'hydrate de carbone, et du quart à la moitié de l'hydrate de carbone qui y est contenu est remplacé par de l'amidon de maïs cru.

7. Utilisation selon la revendication 6, dans laquelle l'amidon de maïs cru est contenu dans une garniture de la barre à croquer ou dans une couche se trouvant sur la barre à croquer et est édulcoré avec un édulcorant qui n'est pas un sucre.

8. Utilisation selon l'une quelconque des revendications précédentes, dans laquelle l'amidon de maïs cru est formulé en doses unitaires de 5 grammes.

9. Utilisation selon l'une quelconque des revendications précédentes, dans laquelle les doses unitaires sont formulées pour une libération prolongée.

10. Utilisation selon l'une quelconque des revendications précédentes, dans laquelle le taux de glucose sanguin est stabilisé à au moins 60 mg/dl pendant au moins 6 heures.
